(19) **Europäisches Patentamt** / **European Patent Office** / **Office européen des brevets**

(11) **EP 3 106 154 A1**

(12) **EUROPEAN PATENT APPLICATION**

(43) Date of publication:
**21.12.2016 Bulletin 2016/51**

(51) Int Cl.:
**A61K 9/70** (2006.01)

(21) Application number: **16174881.9**

(22) Date of filing: **17.06.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.06.2015 JP 2015122404**

(71) Applicant: **NITTO DENKO CORPORATION**
**Osaka (JP)**

(72) Inventors:
- **TACHIKAWA, Yu**
  **Ibaraki-shi, Osaka (JP)**
- **AMEYAMA, Satoshi**
  **Ibaraki-shi, Osaka (JP)**
- **ABE, Eriko**
  **Ibaraki-shi, Osaka (JP)**
- **NAKAMURA, Tetsuya**
  **Ibaraki-shi, Osaka (JP)**
- **URUSHIHARA, Naoko**
  **Ibaraki-shi, Osaka (JP)**
- **ISHIKURA, Jun**
  **Ibaraki-shi, Osaka (JP)**
- **AOYAGI, Kazuhiro**
  **Ibaraki-shi, Osaka (JP)**
- **INOUE, Yoshitaka**
  **Ibaraki-shi, Osaka (JP)**
- **YOKOUCHI, Akira**
  **Ibaraki-shi, Osaka (JP)**
- **TANAKA, Tomoya**
  **Ibaraki-shi, Osaka (JP)**

(74) Representative: **Grünecker Patent- und**
**Rechtsanwälte**
**PartG mbB**
**Leopoldstraße 4**
**80802 München (DE)**

(54) **PATCH PREPARATION**

(57) A patch preparation capable of inhibiting a deviation in pressure-sensitive adhesive properties of a pressure-sensitive adhesive layer even elapsing a long period of time until it is used after production and capable of keeping favorable pressure-sensitive adhesive properties is provided. The patch preparation includes a support and a pressure-sensitive adhesive layer formed on one surface of the support, and the pressure-sensitive adhesive layer includes: (A) a polymer prepared by copolymerizing monomer components including a hydroxyl group-containing monomer and an alkyl (meth)acrylate monomer; (B) a polymer prepared by copolymerizing monomer components including a methyl methacrylate monomer and a butyl methacrylate monomer; and (C) a basic drug, provided that bisoprolol and a salt thereof are excluded.

**Description**

TECHNICAL FIELD

An aspect of the present invention relates to a patch preparation.

BACKGROUND ART

A patch preparation to be adhered to a skin includes a pressure-sensitive adhesive layer(s) formed on one or both surfaces of a support and is intended to intracorporeally administer a drug from the skin via the pressure-sensitive adhesive layer to prevent or treat a disease locally or in a whole body.

Such a patch preparation is required to exhibit thorough pressure-sensitive adhesive properties on adhering to a skin and to be able to be peeled and removed from the skin without causing contamination of the skin surface after the use (for example, occurrence of adhesive deposit, tackiness, etc.). In addition, the patch preparation is desirably low in irritation to the skin.

Patent Document 1 discloses a patch using an acrylic pressure-sensitive adhesive obtained by polymerizing an alkyl (meth)acrylate with a monomer that is copolymerizable with the alkyl (meth)acrylate and does not contain any of a carboxyl group and a sulfo group. Furthermore, in the patch of Patent Document 1, a pressure-sensitive adhesive layer thereof includes an organic liquid component and is crosslinkable. It is mentioned that the patch is low in irritation to the skin and has a soft feeling while having a sufficient cohesive force such that the adhesive deposit is not occurred when the patch is peeled. However, when the pressure-sensitive adhesive disclosed in Patent Document 1 which does not have any of a carboxyl group and a sulfo group is adhered to a skin surface over a long period of time or adhered to a skin surface with large movement, the pressure-sensitive adhesive possibly drops out from the skin, and thus, more improvements of adhesive properties are desired.

Patent Document 2 discloses a patch including a crosslinked product of a copolymer of an alkyl (meth)acrylate, or a mixture of an alkyl (meth)acrylate and an alkoxyalkyl (meth)acrylate, with a monomer containing a carboxyl group and/or a hydroxyl group in a pressure-sensitive adhesive layer. However, in the pressure-sensitive adhesive layer in the above-described patch, in a case where a copolymer having a carboxyl group is contained and a drug has basicity, there is a concern that the carboxyl group is affected by an action of the basic drug to disturb release of the drug from the patch, thereby lowering a utilization rate of the drug.

Meanwhile, in a case where the pressure-sensitive adhesive layer contains a copolymer having a hydroxyl group, not only good adhesiveness to the skin is exhibited, but also there is no concern that the release of the basic drug from the patch preparation is disturbed. However, the hydroxyl group is possibly affected by an action of the basic drug, thereby causing a deviation in pressure-sensitive adhesive properties, such as an increase of a holding power during preservation, etc.

CITATION LIST

PATENT LITERATURE

Patent Document 1: JP-A-2003-313122
Patent Document 2: JP-A-H04-150865

SUMMARY OF INVENTION

TECHNICAL PROBLEM

Under the foregoing circumstances, the present invention has been found, and in an aspect of the present invention, a problem to be solved is to provide a patch preparation capable of inhibiting a deviation in pressure-sensitive adhesive properties of a pressure-sensitive adhesive layer even elapsing a long period of time until it is used after production and capable of keeping favorable pressure-sensitive adhesive properties.

SOLUTION TO PROBLEM

In order to solve the foregoing problem, the present inventors made extensive and intensive investigations. As a result, it has been found that by incorporating a polymer prepared by copolymerizing monomer components including a hydroxyl group-containing monomer and an alkyl (meth)acrylate monomer and a polymer prepared by copolymerizing monomer components including a methyl methacrylate monomer and a butyl methacrylate monomer into a pressure-sensitive adhesive layer containing a basic drug, a deviation in pressure-sensitive adhesive properties during preservation after production can be suppressed, and favorable pressure-sensitive adhesive properties even elapsing a long period of time until it is used after production can be kept, leading to accomplishment of the present invention.

That is, an aspect of the present invention provides the followings.

[1] A patch preparation comprising a support and a pressure-sensitive adhesive layer formed on one surface of the support, the pressure-sensitive adhesive layer containing:

(A) a polymer prepared by copolymerizing monomer components including a hydroxyl group-containing monomer and an alkyl (meth)acrylate monomer;
(B) a polymer prepared by copolymerizing monomer components including a methyl methacrylate monomer and a butyl methacrylate monomer; and
(C) a basic drug, provided that bisoprolol and a salt thereof are excluded.

[2] The patch preparation according to [], wherein the pressure-sensitive adhesive layer further contains an organic liquid component.
[3] The patch preparation according to [1] or [2], wherein a content ratio of the component (A) to the component (B) which are contained in the pressure-sensitive adhesive layer (component (A)/component (B)) is 1/0.05 to 1/1.

ADVANTAGEOUS EFFECTS OF INVENTION

The patch preparation in an aspect of the present invention is small in a deviation in pressure-sensitive adhesive properties during preservation, is able to keep favorable pressure-sensitive adhesive properties even elapsing a long period of time until it is used after production, and has high skin permeability of a drug.

DESCRIPTION OF EMBODIMENTS

An aspect of the present invention is hereunder described in more detail along suitable embodiments thereof.
The patch preparation in an aspect of the present invention is provided as a transdermal absorption type preparation, and specifically, it is provided as a matrix type patch preparation, a reservoir type patch preparation, or the like.
The support in the patch preparation in an aspect of the present invention is not particularly limited, and any support may be used so long as the pressure-sensitive adhesive layer can be formed and holed on one surface thereof. Specific examples thereof include various plastic films such as a polyester, a polyamide (nylon), polyethylene, polypropylene, polyvinyl chloride, polyvinylidene chloride (trade name: SARAN, etc.), an ionomer resin (trade name: SURLYN, etc.), polytetrafluoroethylene, an ethylene-ethyl acrylate copolymer, an ethylene-vinyl alcohol copolymer (trade name: EVAL, etc.), etc., various metal foils, and the like. They may be used alone or as a laminate thereof. In order to enhance an anchoring force with the pressure-sensitive adhesive layer to be laminated, the surface of the support may be subjected to a treatment such as an undercoating agent layer, a corona discharge treatment, a plasma irradiation treatment, a primer treatment, or the like. Furthermore, it is preferred to use a support on which a porous sheet is laminated on the side of the pressure-sensitive adhesive layer forming surface of the support. As the porous sheet in this case, practical examples thereof include paper, a nonwoven fabric, a woven fabric, a knitted fabric, a perforated plastic sheet, and the like. Of those, it is preferred to use paper, a nonwoven fabric, or a woven fabric from the viewpoints of a use feeling during adhesion, adhesion operability, and the like.
A thickness of the support in the above-described patch preparation is preferably 10 to 200 $\mu$m from the standpoints of improvement of anchoring, flexibility and adhesion operability of a pressure-sensitive adhesive tape or the whole of a transdermal absorption type preparation, and the like. In the case of a thin patch preparation, a support having a thickness of from 10 to 100 $\mu$m is adopted. In addition, in the case of using a woven fabric or a nonwoven fabric as the porous sheet, its basis weight may be set to 5 to 30 g/m$^2$, and preferably 6 to 15 g/m$^2$. In an aspect of the present invention, examples of the most suitable support include a laminated film made of a polyester film having a thickness of 1.5 to 6 $\mu$m and a polyester-made nonwoven fabric having a basis weight of 6 to 12 $\mu$g/m$^2$.
In the patch preparation in an aspect of the present invention, in order to protect a pressure-sensitive adhesive surface of the pressure-sensitive adhesive layer until it is used, it is preferred to laminate a release liner to the pressure-sensitive adhesive surface. The release liner is not particularly limited so long as it is thoroughly light and is able to ensure releasability. Specific examples thereof include films of a polyester, polyvinyl chloride, polyvinylidene chloride, polyethylene terephthalate, etc.; papers such as fine paper, glassine paper etc.; laminate films made of fine paper, glassine paper, etc. and a polyolefin; and the like, each being subjected to a release treatment by applying a silicone resin, a fluorine resin or the like to a surface coming into contact with the pressure-sensitive adhesive layer. A thickness of the release linear is typically 10 to 200 $\mu$m, and preferably 25 to 100 $\mu$m.

The polymer as the component (A) that is included in the pressure-sensitive adhesive layer, as prepared by copolymerizing the monomer components including a hydroxyl group-containing monomer and an alkyl (meth)acrylate monomer, is not particularly limited so long as the alkyl (meth)acrylate monomer unit is copolymerized in a proportion of 40% by weight or more, preferably in a proportion of 40 to 90% by mass, and more preferably in a proportion of 50 to 90% by weight per the whole of the polymer, and the hydroxyl group is added in a molecular structure thereof.

In the present specification, the term "(meth)acryl" means both "acryl" and "methacryl".

Although the above-described alkyl (meth)acrylate monomer is not particularly limited, an alkyl (meth)acrylate monomer having an alkyl group having 4 or more carbon atoms is preferred from the viewpoint of adhesiveness. Specific examples thereof include alkyl (meth)acrylate monomers in which the alkyl group thereof is a linear alkyl group or a branched alkyl group each having 4 to 13 carbon atoms, such as butyl, pentyl, hexyl, heptyl, octyl, 2-ethylhexyl, nonyl, decyl, undecyl, dodecyl, tridecyl, etc. Above all, 2-ethylhexyl acrylate is preferred. The alkyl (meth)acrylate monomers may be used alone or in combination of two or more kinds thereof.

The hydroxyl group-containing monomer is not particularly limited, and a hydroxyl group-containing monomer having at least one unsaturated double bond participating in a copolymerization reaction in a molecule thereof may be used. Examples thereof include an N-hydroxyalkyl (meth)acrylamide, hydroxyethyl (meth)acrylate, hydroxypropyl (meth)acrylate, and the like, and an N-hydroxyalkyl acrylamide, hydroxyethyl acrylate, and hydroxypropyl acrylate are preferred. Here, the N-hydroxyalkyl (meth)acrylamide is preferably an N-hydroxyalkyl($C_{1-4}$) (meth)acrylamide in which a carbon number of the alkyl group is 1 to 4, and is more preferably an N-hydroxyalkyl($C_{2-4}$) (meth)acrylamide in which a carbon number of the alkyl group is 2 to 4. The alkyl group in the hydroxyalkyl group may be either linear or branched. Examples of the N-hydroxyalkyl (meth)acrylamide include N-(2-hydroxylethyl) acrylamide, N-(2-hydroxyethyl) methacrylamide, N-(2-hydroxypropyl) acrylamide, N-(2-hydroxypropyl) methacrylamide, N-(1-hydroxypropyl) acrylamide, N-(1-hydroxypropyl) methacrylamide, N-(3-hydroxypropyl) acrylamide, N-(3-hydroxypropyl) methacrylamide, N-(2-hydroxybutyl) acrylamide, N-(2-hydroxybutyl) methacrylamide, N-(3-hydroxybutyl) acrylamide, N-(3-hydroxybutyl) methacrylamide, N-(4-hydroxybutyl) acrylamide, N-(4-hydroxybutyl) methacrylamide, and the like.

Above all, the hydroxy group-containing monomer is preferably N-(2-hydroxyethyl) acrylamide or N-(2-hydroxyethyl) methacrylamide, and is most preferably N-(2-hydroxyethyl) acrylamide.

The hydroxyl group-containing monomers may be used alone or in combination of two or more kinds thereof.

The above-described hydroxy group-containing monomer is contained in an amount of from preferably 1 to 20% by weight, and more preferably I to 15% by weight per the whole of the polymer as the component (A). When the content of the hydroxy group-containing monomer is less than the lower limit, its compatibility with additives such as an organic liquid component, etc., may be poor, so that such additives are possibly unable to be held. When the content of the hydroxy group-containing monomer is more than the upper limit, the hydroxy group-containing monomer is liable to be affected by an action of the basic drug, and a deviation in physical properties with time becomes large.

The polymer as the component (A) may also be a polymer prepared by further copolymerizing the alkyl (meth)acrylate monomer, the hydroxyl group-containing monomer, and a monomer other than the alkyl (meth)acrylate monomer and the hydroxyl group-containing monomer (such a monomer will be hereinafter sometimes referred to as "third monomer"). In a case where the third monomer is contained as the monomer components that constitute the polymer as the component (A), its content is preferably 50% by weight or less, more preferably 1 to 50% by weight, still more preferably 5 to 45% by weight, especially preferably 10 to 45% by weight, and most preferably 15 to 45% by weight in the polymer as the component (A). By using the third monomer in the copolymerization, it is possible to adjust a pressure-sensitive adhesive force and cohesive force of the patch preparation and to adjust solubility and releasability of the drug. When the content of the third monomer in the polymer is more than 50% by weight, the tackiness or pressure-sensitive adhesive force of the resulting patch preparation is possibly deteriorated.

A monomer including nitrogen (N) as a constituent atom (provided that a monomer including, as a constituent atom, nitrogen (N) in the above-described hydroxyl group-containing monomer is excluded) may be used as the third monomer. Examples of such a nitrogen atom-containing monomer include N-vinyl cyclic amides such as N-vinyl-2-pyrrolidone, N-vinyl-2-piperidone, N-vinyl-3-morpholine, N-vinyl-2-caprolactam, N-vinyl-1,3-oxazine-2-on, N-vinyl-3,5-morpholinedione, etc.; and the like. Above all, N-vinyl-2-pyrrolidone is most preferred.

In the polymer as the component (A), its weight average molecular weight (Mw) is preferably in the range of from 1,400,000 to 2,400,000, and more preferably in the range of from 1,600,000 to 2,200,000. When the weight average molecular weight (Mw) falls within such a preferred range, the holding ability of the organic liquid component in the pressure-sensitive adhesive layer, the cohesive force of the pressure-sensitive adhesive layer and the like are high, and favorable pressure-sensitive adhesive properties are obtained.

In an aspect of the present invention, the weight average molecular weight (Mw) is measured by the multi-angle static light scattering detector (MALS) method. Specifically, the weight average molecular weight by MALS may be obtained by continuously injecting a polymer dissolved in tetrahydrofuran and ethanol using a syringe pump while using DAWN DSP, manufactured by Wyatt Technology Corporation, as a detector with respect to the measurement device and performing calculation with a calibration curve of standard polystyrene.

The component (B) that is included in the pressure-sensitive adhesive layer is a polymer prepared by copolymerizing the monomer components including a methyl methacrylate monomer and a butyl methacrylate monomer. A content ratio of the methyl methacrylate monomer to the butyl methacrylate monomer in the polymer is preferably 1/1 to 1/4, and more preferably 1/3 in terms of a weight ratio. A side chain of such a polymer is a neutral group. For this reason, as compared with polymers including a basic group or an acidic group in the side chain, content stability with time of the basic drug included in the pressure-sensitive adhesive layer is obtained.

In the polymer as the component (B), its weight average molecular weight (Mw) is preferably in the range of from 100,000 to 200,000, and more preferably in the range of from 130,000 to 170,000. When the weight average molecular weight (Mw) falls within such a preferred range, the compatibility with the component (A) is good, and favorable pressure-sensitive adhesive properties are obtained.

A content ratio of the component (A) to the component (B) included in the pressure-sensitive adhesive layer (component (A)/component (B)) is preferably 1/0.05 to 1/1, more preferably 1/0.05 to 1/0.8, and especially preferably 1/0.1 to 1/0.4 in terms of a weight ratio. The content ratio of the component (B) to the component (A) (component (B)/component (A)) is smaller than 0.05, a rate of change of the holding power with time may be large, whereas the content ratio (component (B)/component (A)) is larger than 1, on preparing the patch preparation, the component (A) and the component (B) may be mixed heterogeneously, or there is a tendency of occurrence of phase separation with time.

In an aspect of the present invention, the basic drug (component (C)) is a drug having one or two or more basic groups, such as an amino group (for example, primary ($-NH_2$), secondary (-NRH), or tertiary (-NRR')), etc., in a compound, or a salt thereof (exclusive of bisoprolol and salts thereof), and specifically, examples thereof include tulobuterol (TBL), pramipexole (PRA), rivastigmine (LIB), and the like.

The salt of the basic drug may be either an organic acid salt or an inorganic acid salt, and examples thereof include a hydrochloride, a sulfate, an acetate, a phosphate, a carbonate, a mesylate, a tartarate, a citrate, a tosylate, and the like. Two or more salts may also be used in combination, if desired.

A content of the basic drug in the pressure-sensitive adhesive layer may fall within the range where the effects of the basic drug are satisfied, and the pressure-sensitive adhesive properties of the pressure-sensitive adhesive are not impaired. The basic drug is contained in an amount of preferably 0.1 to 40% by weight, and more preferably 1 to 30% by weight based on a total weight of the pressure-sensitive adhesive layer from the viewpoints of an interaction with the polymer including the hydroxyl group-containing monomer and the (meth)acrylic acid ester and the effects of the drug.

In the above-described pressure-sensitive adhesive layer, any component may be used without particular limitations so long as it has an effect for improving an adhesive feeling, and for example, an organic liquid component may be contained. Examples of the organic liquid component include glycols such as ethylene glycol, diethylene glycol, propylene glycol, triethylene glycol, polyethylene glycol, polypropylene glycol, etc.; fats and oils such as olive oil, castor oil, squalene, lanolin, etc.; hydrocarbons such as liquid paraffin, etc.; various surfactants; ethoxylated stearyl alcohol; glycerin esters (monoglycerides, diglycerides, triglycerides, or mixtures thereof) of a long-chain or medium-chain fatty acid, such as oleic acid, caprylic acid, lauric acid, etc.; monohydric alcohol fatty acid esters (monohydric alcohol fatty acid esters composed of a fatty acid having preferably 6 to 22 carbon atoms, and more preferably 12 to 16 carbon atoms and an alcohol having 1 to 20 carbon atoms), such as ethyl laurate, isopropyl myristate, isotridecyl myristate, octyl palmitate, isopropyl palmitate, ethyl oleate, diisopropyl adipate, etc.; higher fatty acids (preferably higher fatty acids having 8 to 22 carbon atoms) such as oleic acid, caprylic acid, etc.; higher alcohols (preferably higher alcohols having 6 to 22 carbon atoms) such as oleyl alcohol, etc.; polyhydric alcohol higher fatty acid esters such as propylene glycol monolaurate, propylene glycol monooleate, propylene glycol monostearate, etc.; citric acid esters, such as triethyl citrate, triethyl acetylcitrate, tributyl citrate, tributyl acetylcitrate, etc.; N-methylpyrrolidone; 1,3-butanediol; and the like. Above all, monohydric alcohol fatty acid esters and polyhydric alcohol fatty acid esters are preferred, and isopropyl myristate and propylene glycol monolaurate (PGML) are especially preferred.

A content of the organic liquid component in the pressure-sensitive adhesive layer is preferably 0.1 to 2.5 parts by weight, more preferably 0.2 to 2.0 parts by weight, and especially preferably 0.3 to 1.5 parts by weight based on 1 part by weight of a total content of the component (A) and the component (B). When the content of the organic liquid component falls within the foregoing range, more favorable adhesiveness to the skin and low irritation to the skin may be obtained.

Not only the above-described organic liquid component but also other arbitrary component(s) may be contained in the pressure-sensitive adhesive layer within the range where the characteristic features in an aspect of the present invention are not impaired. Examples of the arbitrary component(s) include antioxidants such as ascorbic acid, tocopherol acetate, natural vitamin E, dibutylhydroxy toluene, butylhydroxy anisole, etc.; amine-ketone-based anti-aging agents such as 2,6-tert-butyl-4-methylphenol, etc.; aromatic secondary amine-based anti-aging agents such as N,N'-di-2-naphthyl-p-phenylenediamine, etc.; monophenol-based anti-aging agents such as a 2,2,4-trimethyl-1,2-dihydroquinoline polymer, etc.; bisphenol-based anti-aging agents such as 2,2'-methylenebis(4-ethyl-6-tert-butylphenol), etc.; polyphenol-based anti-aging agents such as 2,5-tert-butylhydroquinoline, etc.; fillers such as kaolin, hydrated silicon dioxide, zinc oxide, acrylic acid starch 1000, etc.; softening agents such as polybutene, macrogol 1500, etc.; preservatives such as benzoic acid, sodium benzoate, chlorhexidine hydrochloride, sorbic acid, methyl p-hydroxybenzoate, butyl p-hydroxybenzoate, etc.; colorants such as yellow iron oxide, yellow iron sesquioxide, iron sesquioxide, black iron oxide, carbon black, carmine, β-carotene, copper chlorophyll, Food Color Blue No. 1, Food Color Yellow No. 4, Food Color Red No. 2, licorice extract, etc.; algefacients such as fennel oil, d-camphor, dl-camphor, peppermint oil, d-borneol, l-menthol, etc.; flavors such as spearmint oil, clove oil, vanillin, bergamot oil, lavender oil, etc.; and the like.

A thickness of the pressure-sensitive adhesive layer in an aspect of the present invention is not particularly limited. For example, the thickness of the pressure-sensitive adhesive layer is preferably 5 to 400 μm, more preferably 7 to 200 μm, and still more preferably 10 to 100 μm. So long as the thickness of the pressure-sensitive adhesive layer falls within the foregoing range, favorable pressure-sensitive adhesive properties (for example, adhesive strength) may be realized.

The pressure-sensitive adhesive layer may be formed by giving (typically applying) a coating solution for forming the pressure-sensitive adhesive layer to a support or a release liner and then drying this to remove a solvent. The coating solution for forming the above-described pressure-sensitive adhesive layer includes an appropriate solvent capable of dissolving the pressure-sensitive adhesive layer and the composition in an aspect of the present invention therein.

EXAMPLES

An aspect of the present invention is more specifically described below by reference to Examples. It should be construed that the present invention is not limited to the descriptions of the Examples.

Preparation of polymer (A1):

In a reactor equipped with a cooling tube, a nitrogen gas introducing pipe, a thermometer, a dropping funnel, and a stirrer, 70 parts of 2-ethylhexyl acrylate (hereinafter sometimes represented by "2-EHA"), 5 parts by weight of N-(2-hydroxyethyl) acrylamide (hereinafter sometimes represented by "HEAA"), 25 parts by weight of N-vinyl-2-pyrrolidone (hereinafter sometimes represented by "N-VP"), and 333.3 parts by weight of ethyl acetate as a solvent were added and they were stirred at room temperature for 1 hour while bubbling a nitrogen gas (100 mL/min). Thereafter, the contents in the reactor were heated, and at the moment when the temperature reached 60°C, 0.2 parts by weight of 2,2'-azobisisobutyronitrile (AIBN) as a polymerization initiator was added. The contents were polymerized in a nitrogen gas stream for 6 hours while controlling the temperature so as to keep the temperature of the content at 60°C, and subsequently, the resultant was held at 76°C for 15 hours. According to the solution polymerization of the above-described mode, a pressure-sensitive adhesive composition that is a solution of an acrylic copolymer (2-EHA/HEAA/N-VP = 70/5/25 (weight ratio), Mw: 2,200,000) was obtained.

Preparation of polymer (A-2):

A polymer (A-2) was prepared in the same manner as in the polymer (A-1), except that the amounts of 2-EHA, HEAA, and N-VP were changed to 55 parts by weight, 5 parts by weight, and 40 parts by weight, respectively. The Mw was 2,000,000.

Preparation of polymer (A-3):

A polymer (A-3) was prepared in the same manner as in the polymer (A-1), except that 70 parts by weight of 2-EHA, 10 parts by weight of hydroxyethyl acrylate (hereinafter sometimes represented by "HEA"), and 20 parts by weight of N-VP were used as monomers.

As the component (A), the polymer (A1), the polymer (A2) (trade name: DT-87-2287, manufactured by Henkel), or the polymer (A3) was used.

As the component (B), a copolymer having a content ratio of a methyl methacrylate monomer to a butyl methacrylate monomer of 1/3 in terms of a weight ratio (trade name: PLASTOID B (manufactured by Rohm Pharma, weight average molecular weight: 150,000): polymer (B1)) was used.

As the component (C), tulobuterol (TBL), pramipexole (PRA), or rivastigmine (LIB) was used. All of these compounds were used in a free form.

As the organic liquid component, isopropyl myristate (IPM) or propylene glycol monolaurate (PGML) was used.

(Example 1)

52 parts by weight (in terms of solid content) of the polymer (A1) as the component (A), 3 parts by weight of the component (B), 5 parts by weight of tulobuterol as the component (C), and 40 parts by weight of isopropyl myristate as the organic liquid component were mixed, and a suitable amount of ethyl acetate for the concentration adjustment was added thereto, and the contents were stirred to obtain a homogenous pressure-sensitive adhesive solution. The resulting pressure-sensitive adhesive solution was applied in a thickness after drying of 50 $\mu$m on a release treating surface of a polyester-made release sheet having a thickness of 75 $\mu$m, followed by drying at 80°C for 5 minutes to form a pressure-sensitive adhesive layer. Subsequently, a laminate of a polyester film having a thickness of 4 $\mu$m and a polyester-made nonwoven fabric having a basis weight of 12 g/m$^2$ was used as a support, and a nonwoven fabric surface thereof was pressed and adhered to the above-described pressure-sensitive adhesive layer, thereby obtaining a patch preparation in an aspect of the present invention.

(Example 2)

A patch preparation in an aspect of the present invention was obtained in the same manner as in Example 1, except that the amount of the polymer (A1) as the component (A) was changed to 50 parts by weight (in terms of solid content); and the amount of the component (B) was changed to 5 parts by weight.

(Example 3)

A patch preparation in an aspect of the present invention was obtained in the same manner as in Example 1, except that the amount of the polymer (A1) as the component (A) was changed to 43 parts by weight (in terms of solid content); and the amount of the component (B) was changed to 12 parts by weight.

(Example 4)

A patch preparation in an aspect of the present invention was obtained in the same manner as in Example 1, except that the amount of the polymer (A) as the component (A) was changed to 39 parts by weight (in terms of solid content); and the amount of the component (B) was changed to 16 parts by weight.

(Example 5)

A patch preparation in an aspect of the present invention was obtained in the same manner as in Example 1, except that the amount of the polymer (A1) as the component (A) was changed to 43 parts by weight (in terms of solid content); the amount of the component (B) was changed to 12 parts by weight; and the component (C) was changed to 5 parts by weight of pramipexole.

(Example 6)

A patch preparation in an aspect of the present invention was obtained in the same manner as in Example 1, except that the amount of the polymer (A1) as the component (A) was changed to 50 parts by weight (in terms of solid content); the amount of the component (B) was changed to 10 parts by weight; the component (C) was changed to 10 parts by weight of rivastigmine; and the organic liquid component was changed to 30 parts by weight of isopropyl myristate.

(Example 7)

A patch preparation in an aspect of the present invention was obtained in the same manner as in Example 1, except that the component (A) was changed to 67 parts by weight (in terms of solid content) of the polymer (A2); the amount of the component (B) was changed to 18 parts by weight; the amount of the component (C) was changed to 5 parts by weight; and the organic liquid component was changed to 10 parts by weight of propylene glycol monolaurate.

(Example 8)

A patch preparation in an aspect of the present invention was obtained in the same manner as in Example 1, except that the component (A) was changed to 43 parts by weight (in terms of solid content) of the polymer (A3); and the amount of the component (B) was changed to 12 parts by weight.

(Comparative Example 1)

A patch preparation was obtained in the same manner as in Example 1, except that the amount of the polymer (A1) as the component (A) was changed to 55 parts by weight (in terms of solid content); and the amount of the component (B) was changed to 0 part by weight.

(Comparative Example 2)

A patch preparation was obtained in the same manner as in Example I, except that the amount of the polymer (A1) as the component (A) was changed to 55 parts by weight (in terms of solid content); the amount of the component (B) was changed to 0 part by weight; and the component (C) was changed to 5 parts by weight of pramipexole.

(Comparative Example 3)

A patch preparation was obtained in the same manner as in Example 1, except that the amount of the polymer (A1) as the component (A) was changed to 60 parts by weight (in terms of solid content); the amount of the component (B) was changed to 0 part by weight; the component (C) was changed to 10 parts by weight of rivastigmine; and the organic liquid component was changed to 30 parts by weight of isopropyl myristate.

(Comparative Example 4)

A patch preparation was obtained in the same manner as in Example 1, except that the component (A) was changed to 85 parts by weight (in terms of solid content) of the polymer (A2); the amount of the component (B) was changed to 0 part by weight; and the organic liquid component was changed to 10 parts by weight of propylene glycol monolaurate.

(Comparative Example 5)

A patch preparation was obtained in the same manner as in Example 1, except that the component (A) was changed to 55 parts by weight (in terms of solid content) of the polymer (A3); and the amount of the component (B) was changed to 0 part by weight.

<Measurement of holding power>

A sample prepared by cutting each of the patch preparations into a size of 10 mm in width and 50 mm in length was used as a test piece. The release liner of the test piece was removed, and the pressure-sensitive adhesive layer of the test piece was adhered to one end of a test plate (bakelite plate) in conformity with JIS Z0237:2009 and pressed with a rubber roller of 2 kg by one reciprocation. After press adhesion, the resultant was allowed to stand at 40°C for 30 minutes. Thereafter, a load of 150 g was applied, and a time until falling was reached was determined and defined as a holding power (sec).

With respect to each of the patch preparations of the Examples and Comparative Examples, the holding power at the time of production (just after the production) and the holding power after standing at 50°C for 2 weeks after the production were measured, an index of rate of change of holding power, which is defined by the following formula on the basis of a rate of change of holding power (%) calculated according to the following formula, was determined and shown in Tables 1 to 5. The index of rate of change of holding power is an index of maintainability of pressure-sensitive adhesive properties (holding power) of the patch preparation, and the smaller the value of this index of rate of change of holding power is, the more excellent the maintainability of the holding power is.

- $$\text{Rate of change of holding power (\%)} = ((\text{Holding power}_{(50°C,\ 2W)} - (\text{Holding power}_{(just\ after\ production)}))/(\text{Holding power}_{(just\ after\ production)}) \times 100$$

- $$\text{Index of rate of change of holding power} = (\text{Rate of change of holding power of Example or Comparative Example})/(\text{Rate of change of holding power of Comparative Example}) \times 100$$

<Measurement of pressure-sensitive adhesive force >

A sample prepared by cutting each of the patch preparations into a size of 12 mm in width was used as a test piece. The release liner of the test piece was removed, and a pressure-sensitive adhesive force value of the pressure-sensitive adhesive layer was determined in conformity with JIS Z0237:2009 (pressure-sensitive adhesive force by 180° peel method). The test was carried out at room temperature, and a measured value was expressed in terms of a pressure-sensitive adhesive force (N/12 mm).

The following Tables I to 5 show the constitution, pressure-sensitive adhesive force, and index of rate of change of holding power of the pressure-sensitive adhesive layer of each of the patch preparations prepared in the Examples and Comparative Examples. In the tables, the numerical values of the components (A) to (C) and the organic liquid component are represented in terms of parts by weight. The "2W" represents "two weeks".

Table 1

| | Component (A) Polymer (A1) | Component (B) Polymer (B1) | Organic liquid component IPM | Component (C) PRA | Component (C) TBL | Component (C) LIB | Index of rate of change of holding power on the basis of Comparative Example 1 | Pressure-sensitive adhesive force (N/12 mm) At the beginning | Pressure-sensitive adhesive force (N/12 mm) 50°C, 2W |
|---|---|---|---|---|---|---|---|---|---|
| Comparative Example 1 | 55 | - | 40 | - | 5 | - | 100 | 2.40 | 2.42 |
| Example 1 | 52 | 3 | 40 | - | 5 | - | 72 | 2.83 | 2.68 |
| Example 2 | 50 | 5 | 40 | - | 5 | - | 61 | 3.11 | 2.73 |
| Example 3 | 43 | 12 | 40 | - | 5 | - | 19 | 2.77 | 2.84 |
| Example 4 | 39 | 16 | 40 | - | 5 | - | 20 | 3.05 | 3.06 |

Table 2

| | Component (A) | Component (B) | Organic liquid component | Component (C) | | | Index of rate of change of holding power on the basis of Comparative Example 2 | Pressure-sensitive adhesive force (N/12 mm) | |
|---|---|---|---|---|---|---|---|---|---|
| | Polymer (A 1) | Polymer (B1) | IPM | PRA | TBL | LIB | | At the beginning | 50°C, 2W |
| Comparative Example 2 | 55 | - | 40 | 5 | - | - | 100 | - | - |
| Example 5 | 43 | 12 | 40 | 5 | - | - | 15 | - | - |

Table 3

| | Component (A) | Component (B) | Organic liquid component | Component (C) | | | Index of rate of change of holding power on the basis of Comparative Example 3 | Pressure-sensitive adhesive force (N/12 mm) | |
|---|---|---|---|---|---|---|---|---|---|
| | Polymer (A1) | Polymer (B1) | IPM | PRA | TBL | LIB | | At the beginning | 50°C,2W |
| Comparative Example 3 | 60 | - | 30 | - | - | 10 | 100 | 2.50 | 2.36 |
| Example 6 | 50 | 10 | 30 | - | - | 10 | 44 | 3.15 | 2.66 |

Table 4

| | Component (A) | Component (B) | Organic liquid component | Component (C) | | | Index of rate of change of holding power on the basis of Comparative Example 4 | Pressure-sensitive adhesive force (N/12 mm) | |
|---|---|---|---|---|---|---|---|---|---|
| | Polymer (A2) | Polymer (B1) | IPM | PRA | TBL | LIB | | At the beginning | 50°C, 2W |
| Comparative Example 4 | 85 | - | 10 | - | 5 | - | 100 | - | - |
| Example 7 | 67 | 18 | 10 | - | 5 | - | 50 | - | - |

Table 5

| | Component (A) | Component (B) | Organic liquid component | Component (C) | | | Index of rate of change of holding power on the basis of Comparative Example 5 | Pressure-sensitive adhesive force (N/12 mm) | |
| --- | --- | --- | --- | --- | --- | --- | --- | --- | --- |
| | Polymer (A3) | Polymer (B1) | IPM | PRA | TBL | LIB | | At the beginning | 50°C, 2W |
| Comparative Example 5 | 55 | - | 40 | - | 5 | - | 100 | 3.10 | 3.01 |
| Example 8 | 43 | 12 | 40 | - | 5 | - | 86 | 2.88 | 2.35 |

While the invention has been described in detail and with reference to specific embodiments thereof, it will be apparent to one skilled in the art that various changes and modifications can be made therein without departing from the spirit and scope thereof.

This application is based on Japanese Patent Application No. 2015-122404 filed on June 17, 2015, the entire subject matter of which is incorporated herein by reference.

**Claims**

1. A patch preparation comprising a support and a pressure-sensitive adhesive layer formed on one surface of the support, the pressure-sensitive adhesive layer containing:

    (A) a polymer prepared by copolymerizing monomer components including a hydroxyl group-containing monomer and an alkyl (meth)acrylate monomer;
    (B) a polymer prepared by copolymerizing monomer components including a methyl methacrylate monomer and a butyl methacrylate monomer; and
    (C) a basic drug, provided that bisoprolol and a salt thereof are excluded.

2. The patch preparation according to claim I, wherein the pressure-sensitive adhesive layer further contains an organic liquid component.

3. The patch preparation according to claim 1 or 2, wherein a content ratio of the component (A) to the component (B) which are contained in the pressure-sensitive adhesive layer (component (A)/component (B)) is 1/0.05 to 1/1.

Europäisches
Patentamt

European
Patent Office

Office européen
des brevets

# EUROPEAN SEARCH REPORT

Application Number

EP 16 17 4881

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 198 28 274 A1 (ROTTAPHARM BV [NL]) 30 December 1999 (1999-12-30) * the whole document * | 1-3 | INV. A61K9/70 |
| X | US 2009/311310 A1 (AMEYAMA SATOSHI [JP] ET AL) 17 December 2009 (2009-12-17) * paragraphs [0001], [0007] * * paragraph [0010] - paragraph [0058] * * paragraph [0073] - paragraph [0082]; examples 1-3; table 1 * * paragraph [0110]; claims 1-3, 6-8 * | 1-3 | |
| X | US 2014/220104 A1 (HWANG YONG-YOUN [KR] ET AL) 7 August 2014 (2014-08-07) * paragraph [0007] - paragraph [0039] * * tables 1, 2 * * claims 1-4, 8, 9 * | 1-3 | |

TECHNICAL FIELDS SEARCHED (IPC)

A61K

The present search report has been drawn up for all claims

| Place of search | Date of completion of the search | Examiner |
|---|---|---|
| The Hague | 2 November 2016 | González Ferreiro, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## ANNEX TO THE EUROPEAN SEARCH REPORT
## ON EUROPEAN PATENT APPLICATION NO.

EP 16 17 4881

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2016

| Patent document cited in search report | | Publication date | Patent family member(s) | | Publication date |
|---|---|---|---|---|---|
| DE 19828274 | A1 | 30-12-1999 | AT | 216876 T | 15-05-2002 |
| | | | AU | 4508799 A | 10-01-2000 |
| | | | DE | 19828274 A1 | 30-12-1999 |
| | | | EP | 1089720 A1 | 11-04-2001 |
| | | | ES | 2178445 T3 | 16-12-2002 |
| | | | PT | 1089720 E | 31-10-2002 |
| | | | WO | 9966907 A1 | 29-12-1999 |
| US 2009311310 | A1 | 17-12-2009 | CA | 2668978 A1 | 16-12-2009 |
| | | | CN | 101606925 A | 23-12-2009 |
| | | | EP | 2135905 A2 | 23-12-2009 |
| | | | JP | 5368168 B2 | 18-12-2013 |
| | | | JP | 2010024224 A | 04-02-2010 |
| | | | KR | 20090130834 A | 24-12-2009 |
| | | | US | 2009311310 A1 | 17-12-2009 |
| US 2014220104 | A1 | 07-08-2014 | EP | 2711002 A2 | 26-03-2014 |
| | | | JP | 2014513719 A | 05-06-2014 |
| | | | US | 2014220104 A1 | 07-08-2014 |
| | | | WO | 2012161489 A2 | 29-11-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003313122 A
- JP H04150865 A
- JP 2015122404 A